# EUROPEAN PATENT APPLICATION

(11) **EP 3 834 825 A1**
(43) Date of publication of application: **16.06.2021**
(21) Application number: 18929739.3
(22) Date of filing: 29.08.2018
(51) Int. Cl.: A61K 31/255, A61K 9/20, A61K 47/40, A61P 7/00, A61P 35/00

(54) **BUSULFAN COMPOSITION, PREPARATION METHOD THEREFOR AND APPLICATION THEREOF**

(30) Priority: 07.08.2018 CN 201810888451
(71) Applicant: Jiangsu Linghang Biological Technology Co., Ltd., Nanjing, Jiangsu 210038 (CN)
(72) Inventor: WANG, Gang, Nanjing, Jiangsu 210038 (CN); DONG, Xiangyu, Nanjing, Jiangsu 210038 (CN)
(74) Representative: Cabinet Beau de Loménie
(86) International application number: PCT/CN2018/102925
(87) International publication number: WO 2020/029345

(57) **Abstract**

Disclosed in the present invention are a busulfan composition, a preparation method therefor and an application thereof. The busulfan composition comprises the following substances in weight ratio: busulfan and cyclodextrin with a weight ratio of 1-20:100-2000. The busulfan composition is preferably prepared by the following method: dissolving busulfan using an organic solvent to prepare a busulfan solution having a concentration of 1-20 mg/mL; dissolving SBE-β-cyclodextrin using water for injection to prepare a 10-40% (w/v) SBE-beta-cyclodextrin aqueous solution; mixing the two solutions in nitrogen atmosphere, stirring for 1 hour, and then removing the organic solvent; and performing filtering and freeze drying. The present invention can be used in tablets and injections. Studies show that the present invention can significantly improve the stability of busulfan. The present invention has lower irritation in injection, can be used for clinical injection, and improves the medication safety while ensuring product stability.

## Description

### Technical Field of the Invention

The present invention belongs to the field of pharmaceutical preparations, and relates to a Busulfan composition and preparation methods and applications thereof.

### Background of the Invention

Busulfan is a bifunctional alkylating agent of dimethyl sulfonate, which was first discovered in 1953. In terms of mechanism of action, Busulfan is a cytotoxic anti-tumor drug. After being administrated to the human body, its sulfonate group alkylates with guanine in tumor cells, which interferes with the formation of genetic material in tumor cells and thus inhibits the growth of tumor cells. Busulfan works well for proliferative diseases of blood and bone marrow, especially well for chronic myelogenous leukemia, primary thrombocytosis, polycythemia vera, primary myelofibrosis and other diseases. However, due to its poor solubility, especially its water solubility, Busulfan is difficult to be absorbed after oral administration, and the dosage for oral administration is high. Meanwhile, the great individual difference in drug absorption makes it difficult to accurately control the dosage. Therefore, the commercially available Busulfan products are mainly injection.

At present, the commercially available Busulfan injection is only manufactured by Ben Venue laboratories. Inc, and sold in China after sub-packaging. The injection is prepared from a mixed solution of N,N-dimethylacetamide and polyethylene glycol 400 in water in a certain proportion according to the process disclosed in Patent US5559148 by Andersson, Borje S. et al. However, the reproductive toxicity of N,N-dimethylacetamide and the corrosiveness of plastic products pose a great risk to the clinical application of Busulfan products. In addition, the commercially available Busulfan injection will be crystallized during storage, and the crystals cannot be dissolved by shaking. It is stated in the product specification that the product should be observed for crystallization before use. Crystallization will first lead to the inability to determine the use dosage of drugs, and also block the syringe needle or the capillaries of patients, bringing great inconvenience to clinical medication and increasing the risk of use. Crystallized Busulfan injection, which cannot be used clinically, has to be destroyed, which greatly increases the medication cost. It has been reported that hard plastic wares made of ABS (a polymer composed of allylidene nitrile, butadiene and styrene) will be decomposed when coming into contact with dimethylacetamide, an excipient of commercially available Busulfan preparations. To prevent the extraction of the plasticizer DEHP (bis [2-ethylethyl] phthalate) from polyvinyl chloride (PVC) containers by the commercially available Busulfan preparations, the dilute solution of the commercially available Busulfan preparations should be prepared in large-volume sterilized containers free of DEHP, such as glass or polyolefin containers. DEHP-free equipment should also be used in the infusion of the commercially available Busulfan preparations to patients.

In addition, dimethylacetamide may also affect fertility. Administration of 0.45 g/kg/d of DMA (equivalent to 44% of the recommended daily dose of dimethylacetamide in a commercially available Busulfan preparation in mg/m²) to rats for 9 consecutive days may result in a significant reduction of spermatogenesis in rats. A one-time subcutaneous injection of 2.2 g/kg of dimethylacetamide (equivalent to 27% of dimethylacetamide in the Busulfan preparation in mg/m²) for four days after artificial insemination may result in the termination of pregnancy in 100% hamsters under test.

To date, there has not been any research or report on Busulfan injection that meets the current Busulfan drug standard and also shows safety of medication and stability in long-term storage. Therefore, it is an urgent problem to prepare a stable Busulfan injection that is easy for long-term storage from the perspectives of curative effect, drug safety and production.

### Summary of the Invention

An objective of the present invention is to provide a Busulfan composition to overcome the above deficiencies of the prior art.

Another objective of the present invention is to provide preparation methods of the Busulfan composition.

Yet another objective of the present invention is to provide applications of the Busulfan composition.

The objectives of the present invention may be achieved by technical solutions as follows. A Busulfan composition is provided, containing Busulfan and cyclodextrin in a weight ratio of 1-20: 100-2000, preferably 1:30-100, and more preferably 1:75.

The cyclodextrin is preferably any one of sulfobutyl ether-β-cyclodextrin, hydroxypropyl-P-cyclodextrin, methyl-P-cyclodextrin and sulfobutyl-hydroxypropyl-β-cyclodextrin, and more preferably SBE-β-cyclodextrin.

The Busulfan composition is preferably prepared by the following steps: dissolving Busulfan in an organic solvent to obtain a Busulfan solution with a concentration of 1-20 mg/mL; dissolving SBE-β-cyclodextrin with water for injection to obtain 10-40% (w/v) aqueous solution of SBE-beta-cyclodextrin; mixing the two solutions under nitrogen atmosphere, and stirring the mixed solution for 1 hour to remove the organic solvent; and filtering and freeze drying the mixed solution.

Preferably, the Busulfan is dissolved in acetone at a temperature not exceeding 35°C to obtain a Busulfan acetone solution with a concentration of 1-20 mg/mL.

The organic solvent for dissolving Busulfan is preferably acetone, methanol, ethanol and propylene glycol, more preferably acetone.

Further, the composition is preferably composed of the following components: 60 mg of Busulfan, 4.5 g of SBE-β-cyclodextrin, 10 mL acetone and 20 mL water for injection.

Further, the composition is prepared by following steps of: dissolving 60 mg of Busulfan raw material in 10 mL acetone at a temperature not exceeding 35°C; dissolving 4.5 g of sulfobutyl ether-β-cyclodextrin in 20 mL water for injection; mixing the two solutions, introducing nitrogen, stirring the mixed solution for 1 hour under nitrogen atmosphere to remove the organic solvent, filtering the remaining solution by a 0.22 µm microporous membrane, freeze drying and packaging the solution.

The present invention provides preparation methods of the Busulfan composition, including:
Method 1: dissolving Busulfan in an organic solvent to obtain a Busulfan solution with a concentration of 1-20 mg/mL; dissolving SBE-β-cyclodextrin with water for injection to obtain 10-40% (w/v) aqueous solution of SBE-beta-cyclodextrin; mixing the two solutions under nitrogen atmosphere by stirring for 1 hour, and removing the organic solvent; and filtering and freeze drying the mixed solution; or
Method 2: adding the Busulfan raw material and sulfobutyl ether-β-cyclodextrin to water for injection, and then adding an organic solvent; introducing nitrogen, stirring the mixed solution for 4 hours under nitrogen atmosphere to remove the organic solvent, filtering the remaining solution by a 0.22 µm microporous membrane, freeze drying, and packaging the solution, wherein the mass-to-volume ratio of sulfobutyl ether-P-cyclodextrin to water for injection is 1 g : 10-40 mL, and that of Busulfan to organic solvent is 1-20 mg : 1 mL.

The organic solvent for dissolving the Busulfan is selected from acetone, methanol, ethanol or propylene glycol, more preferably acetone in Method 1 and ethanol in Method 2.

The present invention provides applications of the Busulfan composition in the preparation of Busulfan tablets or injections.

The present invention has the following beneficial effects.

The Busulfan composition in this patent has the following advantages.
1. It is free of dimethylacetamide, which reduces the risk of hallucination and infertility caused by dimethylacetamide. Because of being free of dimethylacetamide, the composition in present invention does not contain any components capable of degrading plastic containers. Therefore, there is no risk of DEHP in the filling containers, or in utensils used during extraction and preparation.
2. The composition is stable without crystallization during dilution, thus reducing the use cost.
3. The composition is still stable without crystallization within 12 hours after being diluted, thus improving the use safety.

The stability of the composition is improved, the storage cost is reduced, and the efficacy and safety thereof are guaranteed.

### Brief Description of the Drawings

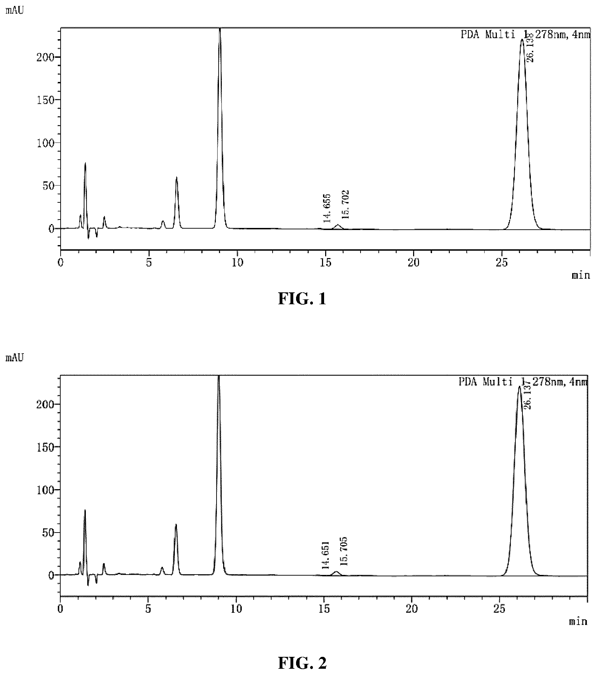

FIG. 1 is an injection liquid chromatogram of a Busulfan composition prepared according to Embodiment 1 after being stored at -20°C for 30 days; and
FIG. 2 is an injection liquid chromatogram of the Busulfan composition prepared according to Embodiment 1 after being stored at 40°C for 30 days.

### Detailed Description of the Invention

Embodiment 1: Preparation of a Busulfan composition according to the present invention 60 mg of the Busulfan raw material is dissolved in 10 mL acetone at a temperature not exceeding 35°C, and 4.5 g of sulfobutyl ether-β-cyclodextrin is dissolved in 20 mL water for injection. The two solutions are mixed, nitrogen is introduced, the mixed solution is stirred for 1 hour under nitrogen atmosphere to remove the organic solvent, and the remaining solution is filtered by a 0.22 µm microporous membrane, freeze-dried and packaged.
Embodiment 2: Preparation of a Busulfan composition according to the present invention 60 mg of the Busulfan raw material is dissolved in 10 mL acetone at a temperature not exceeding 35°C, and 6.0 g of sulfobutyl ether-β-cyclodextrin is dissolved in 20 mL water for injection. The two solutions are mixed, nitrogen is introduced, the mixed solution is stirred for 1 hour under nitrogen atmosphere to remove the organic solvent, and the remaining solution is filtered by a 0.22 µm microporous membrane, freeze-dried and packaged.
Embodiment 3: Preparation of a Busulfan composition according to the present invention 60 mg of the Busulfan raw material is dissolved in 10 mL acetone at a temperature not exceeding 35°C, and 2.0 g of sulfobutyl ether-β-cyclodextrin is dissolved in 10 mL water for injection. The two solutions are mixed, nitrogen is introduced, the mixed solution is stirred for 2.5 hour under nitrogen atmosphere to remove the organic solvent, and the remaining solution is filtered by a 0.22 µm microporous membrane, freeze-dried and packaged.
Embodiment 4: Preparation of a Busulfan composition according to the present invention 60 mg of Busulfan raw material and 3.0 g of sulfobutyl ether-β-cyclodextrin are added to 10 mL water for injection, and then 10 mL ethanol is added. Nitrogen is introduced, the mixed solution is stirred for 4 hours under nitrogen atmosphere to remove the organic solvent, and the remaining solution is filtered by a 0.22 µm microporous membrane, freeze-dried and packaged.
Embodiment 5: Preparation of a Busulfan composition according to the present invention 60 mg of Busulfan and 4.5 g of sulfobutyl ether-P-cyclodextrin are added to 20 mL of water for injection, and then 10 mL methanol is added. Nitrogen is introduced, the mixed solution is stirred for 4 hours under nitrogen atmosphere to remove the organic solvent, and the remaining solution is filtered by a 0.22 µm microporous membrane, freeze-dried and packaged.
Embodiment 6: Preparation of a Busulfan composition according to the present invention 60 mg of Busulfan and 6.0g of sulfobutyl ether-β-cyclodextrin are added to 20mL of water for injection, and then 10 mL methanol is added. Nitrogen is introduced, the mixed solution is stirred for 4 hours under nitrogen atmosphere to remove the organic solvent, and the remaining solution is filtered by a 0.22 µm microporous membrane, freeze-dried and packaged.

### Dilution stability test of Busulfan injection according to present invention

The commercially available Busulfan injection will crystallize during storage, and the crystals cannot be dissolved by shaking. Crystallization will first lead to the inability to calculate the dosage of drugs, and also block the syringe needle or the capillaries of patients, bringing great inconvenience to clinical medication and increasing the risk of use. Crystallized Busulfan injection, which cannot be used clinically, has to be destroyed. The Busulfan compositions prepared in Embodiments 1 to 6 are stored for 6 months, and diluted to 50 mL and 500 mL, respectively. By observing the compositions after standing for 4, 12 and 24 hours for crystallization, it can be seen from the results in Table 1 that no crystallization was found within 4 hours, indicating that the composition is excellent in dilution stability.

**Table 1: Observation Results of Dilution Crystallization Test of Busulfan Composition**

| Embodiment | Final volume diluted | Standing for 4h | Standing for 12h | Standing for 24h |
|---|---|---|---|---|
| 1 | 50 | Clear and transparent without crystals | Clear and transparent without crystals | Clear and transparent without crystals |
| 1 | 500 | Clear and transparent without crystals | Clear and transparent without crystals | Clear and transparent without crystals |
| 2 | 50 | Clear and transparent without crystals | Clear and transparent without crystals | Clear and transparent without crystals |
| 2 | 500 | Clear and transparent without crystals | Clear and transparent without crystals | Clear and transparent without crystals |
| 3 | 50 | Clear and transparent without crystals | Clear and transparent without crystals | Clear and transparent without crystals |
| 3 | 500 | Clear and transparent without crystals | Clear and transparent without crystals | Clear and transparent without crystals |
| 4 | 50 | Clear and transparent without crystals | A small amount of crystals | A small amount of crystals |
| 4 | 500 | Clear and transparent without crystals | A small amount of crystals | A small amount of crystals |
| 5 | 50 | Clear and transparent without crystals | A small amount of crystals | A small amount of crystals |
| 5 | 500 | Clear and transparent without crystals | A small amount of crystals | A small amount of crystals |
| 6 | 50 | Clear and transparent without crystals | Clear and transparent without crystals | A small amount of crystals |
| 6 | 500 | Clear and transparent without crystals | Clear and transparent without crystals | A small amount of crystals |

### Stability test of Busulfan composition

In this patent, the dimethylacetamide leading to potential risk is removed from the original drug. The original drug will be crystallized during storage. Once crystallized, its dose cannot be accurately determined. The patented product will not be re-dissolved for crystallization, which improves the reliability.

In the research on the stability of the composition, the content and two main impurities are observed. The impurity content standard is that the total content of impurities should not exceed 3.8% according to the impurity content standard in Pharmacopoeia, and the composition herein is qualified.

The Busulfan composition prepared according to Embodiment 1 is placed at -20°C and 40°C for 30 days to detect the relevant impurities therein and compare the increments of the two main impurities.

### Sample test conditions:

Determination by high performance liquid chromatography (HPLC)
Chromatographic conditions: C₁₈ column (250 mm×4.6 mm, 5um), mobile phase: acetonitrile: water: tetrahydrofuran = 55:25:20 (v:v), flow rate: 1.0 mL/min. Test wavelength: 278 nm. Injection volume: 20 µL. Column temperature: 30°C.

### Data analysis:

In the liquid chromatogram, there are two known impurity peaks with retention time of about 14.6 min and 15.7 min respectively. The chromatographic peak with retention time of about 26.1 min is the main peak of Busulfan. The impurity content standard is that the total content of impurities should not exceed 3.8% according to the impurity content standard in Pharmacopoeia, and the composition herein is qualified. The peak areas are shown in Table 2. The comparison shows that the content of Busulfan is only reduced by 1.2% after 30 days of storage at -20°C and 40°C, indicating that the Busulfan composition has good stability.

**Table 2 Changes in Liquid Phase Peak Area of Impurities and Busulfan of Busulfan Composition after 30 Days of Storage at -20°C and 40°C**

| | -20°C | 40°C |
|---|---|---|
| Peak 1 | 0.113% | 0.937% |
| Peak 2 | 1.285% | 1.656% |
| Peak 3 | 98.602% | 97.407% |

## Claims

1. A Busulfan composition, containing:
Busulfan and cyclodextrin in a weight ratio of 1-20: 100-2000, preferably 1:30-100, and more preferably 1:75.

2. The Busulfan composition according to claim 1, wherein the cyclodextrin is selected from any one of sulfobutyl ether-P-cyclodextrin, hydroxypropyl-P-cyclodextrin, methyl-P-cyclodextrin and sulfobutyl-hydroxypropyl-P-cyclodextrin, preferably SBE-β-cyclodextrin.

3. The Busulfan composition according to claim 1, wherein the Busulfan composition is prepared by the following steps:
dissolving Busulfan in an organic solvent to obtain a Busulfan solution with a concentration of 1-20 mg/mL; dissolving SBE-β-cyclodextrin with water for injection to obtain 10-40% (w/v) aqueous solution of SBE-beta-cyclodextrin; mixing the two solutions under nitrogen atmosphere, and stirring the mixed solution for 1 hour to remove the organic solvent; and filtering and freeze drying the mixed solution.

4. The Busulfan composition according to claim 3, wherein the Busulfan is dissolved in acetone at a temperature not exceeding 35°C to obtain a Busulfan acetone solution with a concentration of 1-20 mg/mL.

5. The Busulfan composition according to claim 4, wherein the organic solvent for dissolving Busulfan is selected from acetone, methanol, ethanol or propylene glycol, preferably acetone.

6. The Busulfan composition according to any one of claims 1 to 5, wherein the composition is composed of the following components: 60 mg of Busulfan, 4.5 g of SBE-β-cyclodextrin, 10 mL acetone and 20mL water for injection.

7. The Busulfan composition according to claim 6, wherein the composition is prepared by the following steps: dissolving 60mg of Busulfan raw material in 10mL acetone at a temperature not exceeding 35°C; dissolving 4.5g of sulfobutyl ether-β-cyclodextrin in 20mL water for injection; mixing the two solutions, introducing nitrogen, stirring the mixed solution for 1 hour under nitrogen atmosphere to remove the organic solvent, filtering the remaining solution through 0.22 µm microporous membrane, freeze drying and packaging the solution.

8. A preparation method of the Busulfan composition according to any one of claims 1 to 5, comprising following steps of:
Method 1: dissolving Busulfan in an organic solvent to obtain a Busulfan solution with a concentration of 1-20 mg/mL; dissolving SBE-β-cyclodextrin with water for injection to obtain 10-40% (w/v) aqueous solution of SBE-beta-cyclodextrin; mixing the two solutions under nitrogen atmosphere by stirring for 1 hour, and removing the organic solvent; and filtering and freeze drying the mixed solution; or
Method 2: adding the Busulfan raw material and sulfobutyl ether-β-cyclodextrin to water for injection, and then adding an organic solvent; introducing nitrogen, stirring the mixed solution for 4 hours under nitrogen atmosphere to remove the organic solvent, filtering the remaining solution through 0.22 µm microporous membrane, freeze drying, and packaging the solution, wherein the mass-to-volume ratio of sulfobutyl ether-β-cyclodextrin to water for injection is 1 g: 10-40 mL, and that of Busulfan to organic solvent is 1-20 mg: 1 mL.

9. The preparation method according to claim 8, wherein the organic solvent for dissolving the Busulfan is selected from acetone, methanol, ethanol or propylene glycol, preferably acetone in Method 1 and ethanol in Method 2.

10. Application of the Busulfan composition according to any one of claims 1 to 5 in the preparation of Busulfan tablets or injections.
